# EUROPÄISCHE PATENTSCHRIFT

(11) **EP 0 546 428 B1**
(45) Veröffentlichungstag und Bekanntmachung des Hinweises auf die Patenterteilung: **22.01.1997**
(21) Anmeldenummer: 92120483.0
(22) Anmeldetag: 01.12.1992
(51) Int. Cl.: C07D 317/38

(54) **Verfahren zur Herstellung von Ethylenglykolcarbonat**
Method for the preparation of ethylene glycol carbonate
Méthode pour la préparation de carbonate d'éthylène glycol

(30) Priorität: 13.12.1991 DE 4141189
(43) Veröffentlichungstag der Anmeldung: 16.06.1993
(73) Patentinhaber: BAYER AG, 51368 Leverkusen (DE)
(72) Erfinder: Wagner, Paul, Dr., W-4000 Düsseldorf 13 (DE); Buysch, Hans-Josef, Dr., W-4150 Krefeld (DE); Klausener, Alexander, Dr., W-5190 Stolberg (DE); Mais, Franz-Josef, Dr., W-4000 Düsseldorf (DE); Mendoza-Frohn, Christine, Dr., W-4006 Erkrath 2 (DE)

(56) Entgegenhaltungen:
- FETTE, SEIFEN, ANSTRICHMITTEL Bd. 73, Nr. 6, Juni 1971, Hamburg, DE, Seiten 396-399, H. SPRINGMANN

## Beschreibung

Die vorliegende Erfindung betrifft ein Verfahren zur kontinuierlichen katalytischen Herstellung von Ethylenglykolcarbonat (EGC) aus Ethylenoxid (EOX) und Kohlendioxid (CO₂), das gekennzeichnet ist durch eine adiabatische und besonders schonende, energie- und materialsparende Verfahrensweise.

Für die Herstellung von EGC sind viele Vorschläge gemacht worden. Diese beziehen sich in erster Linie auf die Verwendung bestimmter Katalysatoren. Eine ausführliche Darstellung hierzu findet sich in Fette, Seifen, Anstrichmittel 73, (1971), 396 ff. Eine adiabatische Temperaturführung wird in dieser Darstellung als technisch nicht realisierbar beschrieben (loc. cit. 398). Im dort beschriebenen Verfahren werden CO₂ und EOX bei 80 bar und 190 bis oberhalb von 200°C in einem mit Ethylenglykolcarbonat gefüllten Reaktor miteinander umgesetzt und die Reaktionswärme mit Hilfe eines im Gegenstrom zirkulierenden Wärmeträgers abgeführt, der wiederum mit Wasser gekühlt wird. Bei dieser Art der Reaktionsführung erhält man Spitzentemperaturen bis zu 220°C im Reaktor, die produktschädigend wirken, worauf in der angegebenen Publikation ausdrücklich verwiesen wird (S. 397), und die insbesondere bei technischen Großanlagen schwer zu beherrschen sein dürften. Die gesamte Reaktionsenergie wird hierbei ungenutzt abgeführt. Eine adiabatische Temperaturführung ist im Gegensatz hierzu dadurch gekennzeichnet, daß die gesamte freiwerdende Reaktionswärme durch das Reaktionsgemisch selbst aufgenommen wird; dies führt bei exothermen Reaktionen zu einer Erhöhung der Temperatur des Reaktionsgemisches.

Es bestand der Wunsch, ein Verfahren zu entwickeln, das einerseits schädliche Temperaturspitzen vermeidet und andererseits die Reaktionsenergie soweit wie möglich nutzt; eine solche Nutzung kann im Rahmen des erfindungsgemäßen Verfahren selbst, beispielsweise zur destillativen Gewinnung von EGC, oder zur Erzeugung von Heizdampf für andere Verfahren erfolgen.

Es wurde ein Verfahren zur katalytischen Herstellung von Ethylenglykolcarbonat durch Umsetzung von Ethylenoxid und Kohlendioxid in Ethylenglykolcarbonat als Reaktionsmedium bei erhöhter Temperatur und erhöhtem Druck und destillativer Aufarbeitung zur Abtrennung des gebildeten Ethylenglykolcarbonats vom Katalysator gefunden, das dadurch gekennzeichnet ist, daß
a) das Verfahren kontinuierlich und adiabatisch bei einem Druck von 2 bis 200 bar, bevorzugt 5 bis 80 bar, besonders bevorzugt 8 bis 60 bar und innerhalb eines Temperaturbereiches von 110 bis 200°C, bevorzugt 110 bis 190°C, besonders bevorzugt 110 bis 180°C bei einer adiabatischen Temperatursteigerung von 5 bis 80°C durchgeführt wird, wobei die Eingangstemperatur so gewählt wird, daß die adiabatische Temperatursteigerung innerhalb des genannten Temperaturbereiches bleibt,
b) das als Reaktionsmedium dem Reaktor pro Zeiteinheit zulaufende Ethylenglykolcarbonat das 10 bis 120-fache des in dieser Zeiteinheit gebildeten Ethylenglykolcarbonats beträgt,
c) 1,01 bis 1,3 Mol, bevorzugt 1,01 bis 1,25 Mol, besonders bevorzugt 1,01 bis 1,2 Mol Kohlendioxid pro Mol Ethylenoxid eingesetzt werden und an allen Stellen des Reaktors ein Kohlendioxid-Überschuß aufrechterhalten wird,
d) 80 bis 98 Gew.-%, bevorzugt 85 bis 97 Gew.-%, besonders bevorzugt 88 bis 97 Gew.-% des gesamten Reaktionsgemisches an den Eingang des Reaktors zurückgeführt werden und der Rest separat auf Ethylenglykolcarbonat aufgearbeitet wird.

Die Ausgangsprodukte EOX und CO₂ werden in der Regel in einer Reinheit von mindestens 99 % eingesetzt. Es ist jedoch gleichfalls möglich, EOX und CO₂ in geringerer Reinheit einzusetzen, wenn der Rest zu 100 % aus inerten Stoffen, wie beispielsweise Kohlenwasserstoffen, Kohlenmonoxid oder Stickstoff, besteht. Dies gilt besonders für CO₂, das aus verschiedenen Quellen stammen kann, beispielsweise aus natürlichen Quellen oder aus Anlagen zur Erzeugung von Wassergas, Kohlenmonoxid oder Reformern und dementsprechend von geringerer Reinheit ist. Solche Inertgase sind jedoch zweckmäßigerweise in einem Anteil von nicht mehr als 10 Vol.-%, bevorzugt nicht mehr als 5 Vol.-%, besonders bevorzugt nicht mehr als 3 Vol.-% anwesend.

Als Katalysatoren können praktisch alle bisher vorgeschlagenen verwendet werden, wie Alkali- und Erdalkalibromide und -iodide, Guanidine und deren Hydrobromide oder Hydroiodide, Tetraalkylammoniumbromide und -iodide, Phosphoniumbromide und -iodide, Pyridiniumhalogenide, Sulfonium-, Stibonium- und Arsoniumhalogenide, Zink- und Bleihalogenide, Alkylzinnverbindungen oder Gemische aus Alkalihalogeniden mit Halogeniden zweiwertiger Metallionen (FR 1 538 576; BE 872 960; EP 297 647; US 3 535 342; US 2 773 070; US 2 994 705; DE 15 43 555; US 3 535 41 ; BE 798 171; BE 872 959; DE-OS 32 44 456; GB 2 098 985; EP 133 763; EP 180 387; J. Org. Chem. 45 (1980), 3735; Chem. Lett. 1979, 573; DE-OS 41 05 554). In bevorzugter Weise werden als Katalysatoren eingesetzt: Alkalibromide und -iodide, Tetraalkylammoniumbromide und -iodide, Phosphoniumhalogenide, Guanidiniumhalogenide und Gemische aus Alkalihalogeniden mit Halogeniden zweiwertiger Metalle.

Das Verfahren wird im Temperaturbereich von 110 bis 200°C, bevorzugt 110 bis 190°C, besonders bevorzugt 110 bis 180°C durchgeführt. Der Druck für das erfindungsgemäße Verfahren beträgt 2 bis 200 bar, bevorzugt 5 bis 80 bar, besonders bevorzugt 8 bis 60 bar; bei Temperaturen im oberen Teil des angegebenen Bereiches werden auch Drucke im oberen Teil des angegebenen Bereiches benutzt und umgekehrt.

Das erfindungsgemäße Verfahren wird mit adiabatischer Temperaturführung durchgeführt, so daß die adiabatische Temperatursteigerung 5 bis 80° beträgt. Die Eingangstemperatur wird hierbei so gewählt, daß auch bei voller Ausnutzung der gewählten adiabatischen Temperatursteigerung die Obergrenze des angegebenen Temperaturbereiches für das gesamte Verfahren nicht überschritten wird.

Erfindungsgemäß wird an allen Stellen des Reaktors ein CO₂-Überschuß über den anderen Reaktionspartner EOX aufrechterhalten. Hierzu werden 1,01 bis 1,3 Mol, bevorzugt 1,01 bis 1,25 Mol, besonders bevorzugt 1,01 bis 1,2 Mol CO₂ pro Mol EOX eingesetzt.

EGC als Reaktionsmedium liegt stets in großem Überschuß über das neu gebildete EGC vor. So läuft dem Reaktor pro Zeiteinheit das 10 bis 120-fache der in dieser Zeiteinheit gebildete EGC als Umlauf-EGC, das auch den Katalysator enthält, zu. Dieser große Überschuß wird weiterhin dadurch aufrechterhalten, daß 80 bis 98 Gew.-%, bevorzugt 85 bis 97 Gew.-%, besonders bevorzugt 88 bis 97 Gew.-% des gesamten Reaktionsgemisches an den Eingang des Reaktors zurückgeführt werden und lediglich der Rest zu 100 % abgezogen und destillativ auf reines EGC aufgearbeitet wird.

Es ist ein weiteres Kennzeichen des erfindungsgemäßen Verfahrens, daß die durch die adiabatische Temperaturführung entstandene fühlbare Wärme des Reaktionsgemisches genutzt wird. Die wichtigste dieser Nutzungsmöglichkeiten ist hierbei die zur Aufarbeitung des Reaktionsgemisches auf reines EGC. In der Regel ist die erreichte fühlbare Wärme des Reaktionsproduktes jedoch so groß, daß darüber hinaus noch Heizdampf erzeugt werden kann, der anderen (endothermen) Verfahren zugeführt werden kann. In bevorzugter Weise wird hierbei zur Gewinnung der fühlbaren Wärme eine Abkühlung des Reaktionsgemisches um 5 bis 60°C, bevorzugt um 10 bis 50°C, besonders bevorzugt um 10 bis 40°C vorgenommen.

Zur Vermeidung unerwünschter Temperaturspitzen wird im Reaktor eine turbulente Strömung aufrechterhalten.

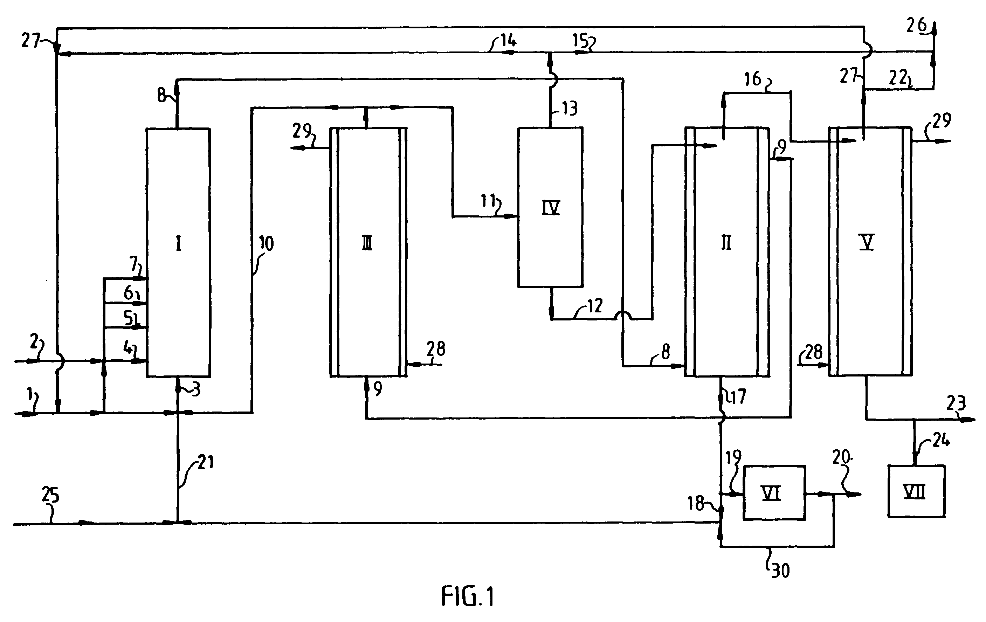

Weitere Einzelheiten des erfindungsgemäßen Verfahren werden im folgenden beschrieben, wozu außerdem auf die beigefügte Fig. 1 verwiesen wird, die beispielhaft eine mögliche Ausführungsform des erfindungsgemäßen Verfahrens zeigt. Selbstverständlich sind auch andere Ausführungsvarianten als die der Fig. 1 möglich.

Der Reaktor (I) ist ein gut isolierter Apparat, beispielsweise ein gut isolierter Rohrreaktor; er enthält Einbauten die eine fortlaufende gute Verteilung der eindosierten Gase EOX und CO₂ gewährleisten und an allen Stellen Turbulenz erzeugen. Solche Einbauten, die dem Fachmann bekannt sind, sind beispielsweise Lochplatten, Loch- und Prallplattenböden, Rohrverteiler (Einstreck- und Ringverteiler), Zweistoffdüsen, Strahldüsen, Düsenboden, technisch übliche Begasungsböden, Sintermetallfritten, geschlossene Gasverteilerböden mit Durchtrittsöffnungen für das EGC, rotierende Begasungsapparate, Impingment Aerator-Elemente (Perrys Chemical Engineers' Handbook 1984 S. 18.57-18.70), Mischerelemente, Blecheinbauten zur Erhöhung der Turbulenz, Segment- und Kreisringumlenkbleche. Diese Einbauten können auch in Kombination miteinander eingesetzt werden. Allgemein sollten solche Einbauten neben einer guten Durchmischung der Strömung (Makrovermischung) und der feinen Gasverteilung einen möglichst großen Anteil hochfrequenter Turbulenzelemente im Dissipationsbereich erzeugen. Bevorzugte Einbauten sind Lochböden, Rohrverteiler, Zweistoffdüsen, Strahldüsen, geschlossene Gasverteiler, Impingment Aerator-Elemente und Mischerelemente.

CO₂ wird im erfindungsgemäßen Verfahren gemäß Ausführungsvariante von Fig. 1 bei (1) und EOX bei (2) zugeführt.

Da es für die hohe Selektivität der erfindungsgemäßen Umsetzung von Bedeutung ist, daß EOX überall im Reaktor auf CO₂ trifft und selber nirgendwo im Überschuß oder auch nur in hohen Konzentrationen auftritt, wird CO₂ in Strömungsrichtung vor dem EOX in den Reaktor eingespeist. Diese vor dem EOX vorgenommene Einspeisung von CO₂ wird zumindest teilweise vorgenommen, während der Rest an CO₂ in einer oder mehreren Teilmengen an weiteren Stellen des Reaktors so eingespeist wird, daß CO₂ stets im Überschuß über das EOX vorhanden ist. In einer bevorzugten Variante wird gemäß Fig. 1 dem in den Reaktor strömenden EGC (3), das bereits den Katalysator enthält, schon vor dem Eintritt in den Reaktor CO₂ vollständig oder wenigstens teilweise zugemischt. In bevorzugter Weise betrifft diese Vorabzumischung von CO₂ die Überschußmenge von CO₂ im Umfang des oben beschrieben über die Menge des EOX hinausgehenden CO₂-Überschusses von 1 bis 30 Mol-%. Erst an einer späteren Eintrittstelle (4) wird EOX zugesetzt, bevorzugt im Gemisch mit CO₂; ein solches Gemisch besteht in besonders bevorzugter Weise aus äquimolaren Mengen EOX und CO₂. In weiterhin bevorzugter Weise wird ein EOX/CO₂-Gemisch an mindestens zwei Einspeisungsstellen in den Reaktor eingespeist. Eine solche mindestens zweite Einspeisungsstelle ist (5), weitere Einspeisungsstellen können (6) und (7) sowie noch weitere sein. Diese eingespeisten Gase werden sodann mit Hilfe der obengenannten Einbauten (Verteilungsorgane) gleichmäßig im Reaktionsmedium verteilt. Die größere Anzahl der Einspeisungsstellen (4), (5), (6), (7) und weitere vermeidet örtliche Überkonzentrationen und örtliche Temperaturspitzen im Reaktor.

Nach dem Abklingen der Hauptreaktion im Reaktor (I) strömt das Reaktionsgemisch (8) zur Nutzung der fühlbaren Wärme in den Heizmantel des Schnellverdampfers (II) und von dort als abgekühltes Reaktionsgemisch (9) zur weiteren Nutzung durch einen Wärmeaustauscher (III) in welchem aus Wasser (28) Heizdampf (29) gewonnen wird. Der Ablauf (8) des Reaktors kann jedoch auch zunächst durch den Wärmetauscher (III) und dann durch den Heizmantel des Schnellverdampfers (II) geführt werden. An die Stelle des Heizdampf erzeugenden Wärmetauschers (III) kann auch ein zu beheizender Reaktor eines völlig anderen Verfahrens treten, dessen Reaktion endotherm verläuft. Man kann jedoch den Ablauf (8) auch vor Eintritt in den Schnellverdampfer (II) in Teilströme, entsprechend (10) und (11), aufteilen.

Für den Reaktorablauf (8) und das bereits etwas abgekühlte Reaktionsgemisch (9) bedeuten die Durchläufe durch (II) und (III) gleichzeitig einen der Nachreaktion dienenden Zeitabschnitt.

Das im Beispiel der Fig. 1 den Wärmeaustauscher (III) verlassende noch weiter abgekühlte Reaktionsgemisch, dessen fühlbare Wärme insgesamt um den obengenannten Betrag von 5 bis 80°C abgenommen hat, wird nunmehr in die Teilströme (10) und (11) aufgeteilt. Der Teilstrom (10) umfaßt in der oben bereits beschriebenen Weise 80 bis 98 Gew.-% des gesamten Ablaufs vom Reaktor (I); der Teilstrom (11) stellt den Rest zu 100 % dar. Selbstverständlich ist es grundsätzlich möglich die Aufteilung von (9) in die Teilströme (10) und (11) an einer anderen Stelle des Verfahrensablaufes vorzunehmen.

Während der Teilstrom (10) zum Reaktoreingang zurückgeführt wird, wird der Teilstrom (11) der Aufarbeitung zugeführt. Hierzu gelangt (11) zunächst in einen Entspannungsbehälter (IV), worin eine Trennung in Flüssigkeit (12) und eine Gasphase (13) erfolgt. Die Gasphase (13) enthält überschüssiges CO₂, gegebenenfalls nicht vollständig umgesetztes EOX und evtl. mit dem CO₂ und dem EOX eingebrachte Inertgase. Je nach dem Anteil an Inertgasen wird die Gasphase (13) in die beiden Teilströme (14) und (15) aufgeteilt. (14) wird wieder in den Reaktor zurückgeführt, während (15) der Abgasentsorgung (26) zugeführt wird. Es ist selbstverständlich, daß bei höheren Inertgasgehalten ein höherer Teil von (13) in Form des Teilstromes (15) ausgeschleust wird und umgekehrt; einfache analytische Bestimmungen und Berechnungen sowie Vorversuche ergeben für den Fachmann ohne Schwierigkeiten das Optimum der Aufteilung der Gasphase (13).

Die in (IV) abgetrennte Flüssigkeit (12) wird nun in den Innenraum des Schnellverdampfers (II) geleitet. Der Schnellverdampfer (II) ist in bevorzugter Weise ein Dünnschichtverdampfer, ein Fallfilmverdampfer, ein Wendelrohrverdampfer oder ein Umlauf- oder Kletterfilmverdampfer. In (II) wird bei einem Vakuum von 2 bis 100 mbar, bevorzugt 8 bis 90 mbar, besonders bevorzugt 10 bis 80 mbar eine destillative Trennung in verdampftes EGC (16) und einen flüssigen Sumpfablauf (17) vorgenommen. Die Energie für diese destillative Trennung entstammt zum einen dem Reaktorablauf (8), der durch den Mantel oder die sonstige Heizeinrichtung des Schnellverdampfers (II) geleitet wird, zum anderen der der flüssigen Phase (12) weiterhin innewohnenden fühlbaren Wärme.

Der Sumpfablauf (17) von (II) wird in die Teilströme (18) und (19) geteilt. Die weitaus größte Menge, beispielsweise 60 bis 98 %, bevorzugt 75 bis 97 %, besonders bevorzugt 90 bis 96 % der Gesamtmenge von (17) lauft als Teilstrom (18) wieder dem Reaktoreingang zu.

Sie enthält weiterhin den Katalysator. Damit schließt sich zudem der Kreislauf des im Reaktorsystem umgewälzten, als Reaktionsmedium dienenden EGC.

Der verbleibende Teilstrom (19) fließt einer Regenerierungsmöglichkeit (VI) für den Katalysator zu. Die Art der Regenerierung des Katalysators ist spezifisch auf den jeweils eingesetzten Katalysator zugeschnitten und ist dem Fachmann, sofern überhaupt eine Katalysatorregenerierung möglich ist, aus der obengenannten Literatur bekannt. Aus der Regenerierung (VI) kann ein Bruchteil von etwa unwirksam gewordenem Katalysator (20) ausgetragen werden. Der regenerierte Katalysator im Gemisch mit EGC (30) wird genau wie der Teilstrom (18) an den Reaktoreingang zurückgeführt. Die Regenerierung kann beispielsweise so durchgeführt werden, daß man Katalysatoren vom Typ der Gemische aus Alkalihalogeniden mit Halogeniden zweiwertiger Metalle, etwa NaBr/ZnBr₂, mit Halogenverbindungen, etwa HBr, behandelt.

Eine Ergänzung von verbrauchtem und ausgetragenem Katalysator durch frischen Katalysator (25) gelangt über (21) in den Reaktor.

Das den Innenraum von (II) verlassende abdestillierte EGC (16) wird in einem weiteren Wärmetauscher (V) unter Dampfgewinnung kondensiert und auf eine Temperatur abgekühlt, die für weitere Umsetzungen, beispielsweise für die Umesterung mit Methanol zu Dimethylcarbonat, erforderlich ist. Ein solches kondensiertes EGC wird bei (23) abgezogen. Selbstverständlich kann das EGC auch weiter abgekühlt werden und als (24) einem Vorratstank (VII) zugeführt werden.

Etwaige gasförmige Anteile von (12), die mit verdampftem (16) nach (V) gebracht wurden, fallen in (V) bei der Kondensation von EGC als gasförmiger, über Kopf abzuziehender Strom an, der in die beiden Teilströme (22) und (27) geteilt wird. (22) wird der Abgasentsorgung (26) zugeführt, während (27) an den Reaktoreingang zurückgeführt wird. Die Aufteilung in die Teilströme (22) und (27) geschieht unter Anlegung ähnlicher Kriterien, wie dies weiter oben für die Teilströme (14) bzw. (15) bereits beschrieben wurde.

(28) ist das den Wärmeaustauschern (III) bzw. (V) zugeführte Wasser, das diesen Apparaten durch Ausnutzung der Verdampfungswärme oder fühlbaren Wärme als gewonnener Heizdampf (29) entnommen werden kann.

### Beispiel

In den bei 15 bar betriebenen Reaktor I gemäß Fig. 1 werden stündlich insgesamt über (1) 0,527 kg CO₂ und über (2) 0,504 kg EOX eindosiert und zwar so, daß je 0,126 kg CO₂ beziehungsweise EOX als Gemisch durch die Leitungen (4) bis (7) fließen und 0,023 kg CO₂ dem über (10) mit einer Temperatur von 140°C zurücklaufenden Reaktionsgemisch von etwa 30,2 kg vor Eintritt in den Reaktor I zugemischt (3) werden. Aus der Entspannung IV strömen weitere 0,03 kg Gas über (13) und (14) in den Reaktor I.

Nach Ablauf der Reaktion verlassen 31,8 kg Reaktionsgemisch mit einer Temperatur von ca. 160°C den Reaktor und fließen als Strom (8) in den Mantel des Schnellverdampfers II, den sie nach Beheizung der darin stattfindenden Destillation mit etwa 151°C verlassen, und als Strom (9) in den Wärmetauscher III, den sie nach Erzeugung von Wasserdampf mit etwa 140°C verlassen, und dann in den Strom (10) von ca. 30,2 kg und (11) von ca. 1,6 kg aufgeteilt werden. (10) kehrt, wie beschrieben, in den Reaktor zurück, während (11) in IV auf 1 bar entspannt wird, wobei die gasförmige Phase (13) von 0,03 kg nach 1 und die flüssige Phase (12) von 1,57 kg in den Schnellverdampfer II geleitet und dort bei etwa 40 mbar in ca. 1,03 kg dampfförmiges EGC (16) und ca. 0,54 kg Sumpf (17) gespalten wird. Das dabei entweichende Restgas von 0,03 kg wird über (22) und (26) in die Abgasentsorgung abgezogen. EGC (16) kondensiert im Wärmetauscher V unter Wasserdampferzeugung und kühlt sich auf eine Temperatur im Bereich von 70 - 110°C ab, wie sie für eine Umesterung, beispielsweise zu Dimethylcarbonat, gewünscht wird (23).

Nach Aufteilung des Sumpfes (17) entsprechend ca. 9:1 fließt die größere Menge von ca. 0,49 kg als Strom (18) und (21) nach I und die kleinere von ca. 0,05 kg (19) in die Regenerierung VI, die sie nach Behandlung mit einer sehr geringen Menge einer Halogenverbindung als (30) wieder verläßt und als (21) zurück in den Reaktor geleitet wird.

## Patentansprüche

1. Verfahren zur katalytischen Herstellung von Ethylenglykolcarbonat durch Umsetzung von Ethylenoxid und Kohlendioxid in Ethylenglykolcarbonat als Reaktionsmedium bei erhöhter Temperatur und erhöhtem Druck und destillative Aufarbeitung zur Abtrennung des gebildeten Ethylenglykolcarbonats vom Katalysator, dadurch gekennzeichnet, daß
a) das Verfahren kontinuierlich und adiabatisch bei einem Druck von 2 bis 200 bar, bevorzugt 5 bis 80 bar, besonders bevorzugt 8 bis 60 bar und innerhalb eines Temperaturbereiches von 110 bis 200°C, bevorzugt 110 bis 190°C, besonders bevorzugt 110 bis 180°C bei einer adiabatischen Temperatursteigerung von 5 bis 80°C durchgeführt wird, wobei die Eingangstemperatur so gewählt wird, daß die adiabatische Temperatursteigerung innerhalb des genannten Temperaturbereiches bleibt,
b) das als Reaktionsmedium dem Reaktor pro Zeiteinheit zulaufende Ethylenglykolcarbonat das 10 bis 120-fache des in dieser Zeiteinheit gebildeten Ethylenglykolcarbonats beträgt,
c) 1,01 bis 1,3 Mol, bevorzugt 1,01 bis 1,25 Mol, besonders bevorzugt 1,01 bis 1,2 Mol Kohlendioxid pro Mol Ethylenoxid eingesetzt werden und an allen Stellen des Reaktors ein Kohlendioxid-Überschuß aufrechterhalten wird,
d) 80 bis 98 Gew.-%, bevorzugt 85 bis 97 Gew.-%, besonders bevorzugt 88 bis 97 Gew.-% des gesamten Reaktionsgemisches an den Eingang des Reaktors zurückgeführt werden und der Rest separat auf Ethylenglykolcarbonat aufgearbeitet wird.

2. Verfahren nach Anspruch 1, dadurch gekennzeichnet, daß im Reaktor eine turbulente Strömung aufrechterhalten wird.

3. Verfahren nach Anspruch 2, dadurch gekennzeichnet, daß der Reaktor Lochböden, Rohrverteiler, Zweistoffdüsen, Strahldüsen, geschlossene Gasverteiler, Impingment Aerator-Elemente, Mischerelemente oder eine Kombination mehrerer von ihnen enthält.

4. Verfahren nach Anspruch 1, dadurch gekennzeichnet, daß zur Erzeugung des Kohlendioxid-Überschusses mindestens ein Teil des Kohlendioxids vor der Eindüsung des Ethylenoxids in das als Reaktionsmedium dienende Ethylenglykolcarbonat eingespeist wird, wobei bevorzugt dieses Kohlendioxid dem Ethylenglykolcarbonat vor Eintritt in den Reaktor zugesetzt wird.

5. Verfahren nach Anspruch 4, dadurch gekennzeichnet, daß Ethylenoxid allein oder als Gemisch mit gegebenenfalls restlichem Kohlendioxid an mindestens zwei aufeinanderfolgenden Stellen so in den Reaktor eingespeist wird, daß stets ein Kohlendioxid-Überschuß besteht.

6. Verfahren nach Anspruch 5, dadurch gekennzeichnet, daß der vorgesehene Überschuß an Kohlendioxid dem Ethylenglykolcarbonat vor dessen Eintritt in den Reaktor zugesetzt wird und das restliche Kohlendioxid als äquimolares Ethylenoxid/Kohlendioxid-Gemisch in den Reaktor eingespeist wird.

7. Verfahren nach Anspruch 1, dadurch gekennzeichnet, daß die erreichte fühlbare Wärme des Reaktionsgemisches außer bei der destillativen Aufarbeitung zu Ethylenglykolcarbonat zusätzlich bei der Erzeugung von Heizdampf oder bei der Durchführung endothermer Verfahren genutzt wird.

8. Verfahren nach Anspruch 7, dadurch gekennzeichnet, daß bei der Gewinnung der fühlbaren Wärme eine Abkühlung des Reaktionsgemisches um 5 bis 80°C, bevorzugt 10 bis 50°C, besonders bevorzugt um 10 bis 40°C eintritt.

9. Verfahren nach Anspruch 7, dadurch gekennzeichnet, daß die destillative Aufarbeitung zu Ethylenglykolcarbonat unter Nutzung der gewonnenen fühlbaren Wärme bei 2 bis 100 mbar, bevorzugt bei 8 bis 90 mbar, besonders bevorzugt bei 10 bis 80 mbar durchgeführt wird.

## Claims

1. Process for the catalytic preparation of ethylene glycol carbonate by reaction of ethylene oxide and carbon dioxide in ethylene glycol carbonate as the reaction medium at elevated temperature and elevated pressure with work-up by distillation to separate the resulting ethylene glycol carbonate from the catalyst, characterised in that
a) the process is carried out continuously and adiabatically at a pressure of 2 to 200 bar, preferably 5 to 80 bar, particularly preferably 8 to 60 bar, and within a temperature range of 110 to 200°C, preferably 110 to 190°C, particularly preferably 110 to 180°C, with an adiabatic temperature increase of 5 to 80°C, the inlet temperature being selected in such a manner that the adia-batic temperature increase remains within the temperature range mentioned,
b) the ethylene glycol carbonate running into the reactor per unit of time as reaction medium is 10 to 120 times the ethylene glycol carbonate formed in this unit of time,
c) 1.01 to 1.3 mol, preferably 1.01 to 1.25 mol, particularly preferably 1.01 to 1.2 mol, of carbon dioxide are used per mole of ethylene oxide and a carbon dioxide excess is maintained at all sites of the reactor,
d) 80 to 98% by weight, preferably 85 to 97% by weight, particularly preferably 88 to 97% by weight, of the total reaction mixture is returned to the inlet of the reactor and the remainder is worked up separately to give ethylene glycol carbonate.

2. Process according to Claim 1, characterised in that a turbulent flow is maintained in the reactor.

3. Process according to Claim 2, characterised in that the reactor contains perforated trays, pipe distributors, two-liquid nozzles, jet nozzles, closed gas distributors, impingement aerator elements, mixer elements or a combination of a plurality thereof.

4. Process according to Claim 1, characterised in that, to produce the carbon dioxide excess, at least some of the carbon dioxide is fed into the ethylene glycol carbonate, acting as reaction medium, prior to the injection of the ethylene oxide, this carbon dioxide preferably being added to the ethylene glycol carbonate prior to entry into the reactor.

5. Process according to Claim 4, characterised in that ethylene oxide is fed into the reactor alone or as a mixture with or without remaining carbon dioxide at at least two sequential sites in such a manner that a carbon dioxide excess always exists.

6. Process according to Claim 5, characterised in that the intended excess of carbon dioxide is added to the ethylene glycol carbonate prior to its entry into the reactor and the remaining carbon dioxide is fed as an equimolar ethylene oxide/carbon dioxide mixture into the reactor.

7. Process according to Claim 1, characterised in that the sensible heat achieved of the reaction mixture, apart from being utilised in the work-up by distillation to give ethylene glycol carbonate, is additionally used to generate heating steam or to carry out endothermal processes.

8. Process according to Claim 7, characterised in that, in the recovery of the sensible heat, a cooling of the reaction mixture by 5 to 80°C, preferably 10 to 50°C, particularly preferably by 10 to 40°C, occurs.

9. Process according to Claim 7, characterised in that the work-up by distillation to give ethylene glycol carbonate with utilisation of the recovered sensible heat is carried out at 2 to 100 mbar, preferably at 8 to 90 mbar, particularly preferably at 10 to 80 mbar.

## Revendications

1. Procédé pour la préparation catalytique de carbonate d'éthylèneglycol par réaction d'oxyde d'éthylène et de dioxyde de carbone dans du carbonate d'éthylèneglycol comme milieu réactionnel, à une température élevée et une pression élevée, et par traitement distillatoire en vue de séparer le carbonate d'éthylèneglycol formé, du catalyseur, lequel procédé est caractérisé en ce que
a) il est réalisé de manière continue et adiabatique à une pression de 2 à 200 bar, de préférence de 5 à 80 bar, une pression de 8 à 60 bar étant particulièrement préférée, et dans les limites d'un intervalle de température de 110 à 200 °C, de préférence de 110 à 190 °C, une température de 110 à 180 °C étant particulièrement préférée, pour une hausse adiabatique de la température de l'ordre de 5 à 80 °C, la température à l'entrée étant choisie de sorte que la hausse adiabatique de la température reste comprise dans l'intervalle de température cité,
b) la quantité du carbonate d'éthylèneglycol amené, par unité de temps, dans le réacteur pour servir de milieu réactionnel est 10 à 120 fois celle du carbonate d'éthylèneglycol formé pendant cette unité de temps,
c) 1,01 à 1,3 mole de dioxyde de carbone, de préférence 1,01 à 1,25 mole, une quantité de 1,01 à 1,2 mole étant particulièrement préférée, est mise en oeuvre par mole d'oxyde d'éthylène et un excédent de dioxyde de carbone est maintenu en tout point du réacteur,
d) 80 à 98 % en poids de la totalité du mélange réactionnel, de préférence 85 à 97 % en poids, une proportion de 88 à 97 % en poids étant particulièrement préférée, sont ramenés à l'entrée du réacteur et le reste est traité séparément pour obtenir du carbonate d'éthylèneglycol.

2. Procédé selon la revendication 1, caractérisé en ce qu'un écoulement turbulent est maintenu dans le réacteur.

3. Procédé selon la revendication 2, caractérisé en ce que le réacteur contient des plateaux à trous, des tubulures de distribution, des tuyères à deux substances, des éjecteurs, des distributeurs fermés de gaz, des éléments d'aération par impact, des éléments mélangeurs ou une combinaison de plusieurs d'entre eux.

4. Procédé selon la revendication 1, caractérisé en ce que pour produire l'excédent de dioxyde de carbone, au moins une partie du dioxyde de carbone est injectée, avant l'oxyde d'éthylène, dans le carbonate d'éthylèneglycol servant de milieu réactionnel, ce dioxyde de carbone étant de préférence ajouté au carbonate d'éthylèneglycol avant son entrée dans le réacteur.

5. Procédé selon la revendication 4, caractérisé en ce que l'oxyde d'éthylène, seul ou en mélange avec d'éventuels restes de dioxyde de carbone, est injecté dans le réacteur en au moins deux points successifs de sorte qu'il existe en permanence un excédent de dioxyde de carbone.

6. Procédé selon la revendication 5, caractérisé en ce que l'excédent prévu de dioxyde de carbone est ajouté au carbonate d'éthylèneglycol avant son entrée dans le réacteur et en ce que le dioxyde de carbone résiduel est injecté dans le réacteur sous la forme d'un mélange équimolaire d'oxyde d'éthylène et de dioxyde de carbone.

7. Procédé selon la revendication 1, caractérisé en ce que la chaleur sensible atteinte dans le mélange réactionnel est utilisée non seulement pour obtenir du carbonate d'éthylèneglycol par distillation, mais aussi pour produire de la vapeur de chauffe ou pour réaliser des procédés endothermiques.

8. Procédé selon la revendication 7, caractérisé en ce que, lors de l'exploitation de la chaleur sensible, la température du mélange réactionnel baisse de 5 à 80 °C, de préférence de 10 à 50 °C, une baisse de 10 à 40 °C étant particulièrement préférée.

9. Procédé selon la revendication 7, caractérisé en ce que la production de carbonate d'éthylèneglycol par distillation est réalisée en utilisant la chaleur sensible produite, à une pression de 2 à 100 mbar, de préférence de 8 à 90 mbar, une pression de 10 à 80 mbar étant particulièrement préférée.
